# EUROPEAN PATENT APPLICATION

(11) **EP 4 793 612 A2**
(43) Date of publication of application: **19.08.2026**
(21) Application number: 26156271.4
(22) Date of filing: 04.02.2026
(51) Int. Cl.: G01L 5/164, G01L 5/165

(54) **INDUCTIVE AND CAPACITIVE SENSORS**

(30) Priority: 12.02.2025 US 202563757613 P; 13.06.2025 US 202563823213 P; 10.12.2025 US 202519414786; 10.12.2025 US 202519414756
(71) Applicant: Analog Devices, Inc., Wilmington, MA 01887 (US)
(72) Inventor: Chen, Baoxing, Wilmington, 01887 (US); Kumar, Deepak, Wilmington, 01887 (US); Zhang, Qian, Wilmington, 01887 (US); Yun, Ruida, Wilmington, 01887 (US)
(74) Representative: Horler, Philip John

(57) **Abstract**

The present disclosure provides inductive and capacitive force sensors. In one aspect, a force sensor cell includes a flexible layer, a first coil arranged on a first side of the flexible layer, and a second coil arranged on a second side of the flexible layer. A force applied to the force sensor cell is configured to change inductive coupling between the first coil and the second coil. Other aspects relate to determining a plurality of directional components of force applied to a pillar of a force sensor and/or capacitive force sensors.

## Description

### BACKGROUND

### INCORPORATION BY REFERENCE TO ANY PRIORITY APPLICATIONS

This application claims the benefit of priority of U.S. Non-Provisional Application No. 19/414,756, filed December 10, 2025, claims the benefit of priority of U.S. Non-Provisional Application No. 19/414,786, filed December 10, 2025, claims the benefit of priority of U.S. Provisional Application No. 63/757,613 filed February 12, 2025 and claims the benefit of priority of U.S. Provisional Application No. 63/823,213, filed June 13, 2025, the disclosures of each of which are hereby incorporated by reference herein in their entireties and for all purposes. Any and all applications for which a foreign or domestic priority claim is identified in the Application Data Sheet as filed with the present application are hereby incorporated by reference under 37 CFR 1.57.

### Technological Field

The disclosed technology relates to sensors and related systems and methods, and particularly, to inductive and capacitive force sensors.

### Description of the Related Technology

Tactile sensors have uses in a variety of applications, including in robotics and treating paralysis. Tactile sensors are configured to sense a force applied to the sensor, which can be used to provide feedback when grasping objects. Tactile sensors can be implemented using different sensing technologies. Magnetic sensors can sense changes in a magnetic field to provide force sensing. Inductive and capacitive sensors can sense changes in inductive or capacitive coupling to provide force sensing.

### SUMMARY

The methods and devices of the described technology each have several aspects, no single one of which is solely responsible for its desirable attributes.

One aspect is a force sensor, comprising: an array of force sensor cells; a plurality of pillars, a first pillar of the plurality of pillars overlapping a force sensor cell of the force sensor cells of the array; and a detection circuit configured to determine a plurality of directional components of a force applied to the first pillar based on output of the force sensor cell.

In some embodiments, the force sensor cell includes: a flexible layer; a first coil arranged on a first side of the flexible layer; and a second coil arranged on a second side of the flexible layer, wherein a force applied to the first pillar is configured to change inductive coupling between the first coil and the second coil.

In some embodiments, the force sensor cell includes: a flexible layer; a first capacitor plate arranged on a first side of the flexible layer; and a second capacitor arranged on a second side of the flexible layer, wherein a force applied to the first pillar is configured to change capacitive coupling between the first capacitor plate and the second capacitor plate.

In some embodiments, the detection circuit is configured to determine a first directional component of the force perpendicular to a plane of the force sensor based on an average of the outputs of the force sensor cell.

In some embodiments, the detection circuit is configured to determine a first directional component of the force parallel to a plane of the force sensor based on a difference in the outputs from at least two sensors of the force sensor cell.

In some embodiments, the plurality of directional components comprise three dimensions of the force.

Another aspect is a robot comprising the force sensor of claim **Error! Reference source not found.** and an end effector, wherein the force sensor is configured for force sensing on the end effector.

Yet another aspect is force sensor cell, comprising: a flexible layer; a first coil arranged on a first side of the flexible layer; and a second coil arranged on a second side of the flexible layer, the second side being opposite to the first side, wherein a force applied to the force sensor cell is configured to change inductive coupling between the first coil and the second coil.

In some embodiments, the first coil is configured to be driven with a first alternating current, and a second alternating current is induced in the second coil due to the inductive coupling with the first alternating current in the first coil.

In some embodiments, the force is configured to change an amplitude of the second alternating current induced in the second coil.

In some embodiments, each of the first coil and the second coil is formed with a plurality of layers.

In some embodiments, the force sensor cell is configured such that an object proximate to the force sensor cell induces a coupling with at least one of the first coil or the second coil, and the coupling changes a parameter of an alternating current of the second coil.

In some embodiments, the force sensor cell further comprises a pillar overlapping the first coil and the second coil, the force being applied to the pillar.

In some embodiments, the force applied to the force sensor cell is configured to increase an amplitude of an alternating current of the second coil, and an object proximate to the force sensor cell is configured to reduce the amplitude of the alternating current of the second coil.

Still yet another aspect is a force sensor, comprising: the force sensor cell; and a detection circuit configured to detect the change in the inductive coupling between the first coil and the second coil.

Another aspect is a robot comprising the force sensor of claim 8 and an end effector, wherein the force sensor is configured for force sensing on the end effector.

Yet another aspect is a method of force sensing, comprising: providing a force sensor cell comprising a flexible layer, a first coil, and a second coil on an opposite side of the flexible layer than the first coil; and detecting force applied to the force sensor cell based on a change in inductive coupling between the first coil and the second coil.

In some embodiments, the method further comprises: driving the first coil with a first alternating current; and detecting a second alternating current induced in the second coil due to the inductive coupling with the first alternating current in the first coil.

In some embodiments, the force change an amplitude of the second alternating current induced in the second coil.

In some embodiments, the force sensor is integrated with an end effector of a robot, and the robot performs the detecting.

### BRIEF DESCRIPTION OF THE DRAWINGS

Embodiments of this disclosure will be described, by way of non-limiting example, with reference to the accompanying drawings.
**FIGs. 1A-1D** illustrate an example tactile sensor cell which can be included in a magnetic tactile sensor in accordance with aspects of this disclosure.
**FIGs. 1E-1F** illustrate another example tactile sensor cell which can be included in a magnetic tactile sensor in accordance with aspects of this disclosure.
**FIG. 2** illustrates a plurality of tactile sensor cells coupled together to form a tactile sensor.
**FIGs. 3A-3B** illustrate another tactile sensor formed from a plurality of tactile sensor cells arranged in an array.
**FIGs. 4A-4B** illustrate another embodiment of a tactile sensor cell in accordance with aspects of this disclosure.
**FIGs. 5A-5B** illustrate another embodiment of a tactile sensor formed from a plurality of tactile sensor cells arranged in an array.
**FIGs. 6A-6F** illustrate embodiments in which the tactile sensor cells can be formed on a single die or on an array of dies.
**FIG. 7** illustrates an embodiment in which the coils and magnetoresistive sensors can be formed on different substrates and then coupled together.
**FIGs. 8A-8H** illustrate additional example tactile sensor cells which can be included in a magnetic tactile sensor in accordance with aspects of this disclosure.
**FIGs. 9A-9B** illustrate still another embodiment of a tactile sensor cell having a multi-turn coil.
**FIGs. 10A-10D** illustrate an example tactile sensor cell which can be used to create a tactile sensor in accordance with aspects of this disclosure.
**FIGs. 11A** and **11B** illustrate an example tactile sensor cell which can be used to create a tactile sensor that can detect proximity of an object in accordance with aspects of this disclosure.
**FIGs. 12A-12C** illustrate another tactile sensor cell which can be used to create a tactile sensor configured to be used for tactile sensing in three dimensions.
**FIGs. 13A-13C** illustrate another tactile sensor cell which can be used to create a tactile sensor configured to be used for tactile sensing in three dimensions.
**FIGs. 14A-14B** illustrate yet another tactile sensor cell which can be used to create a tactile sensor configured to be used for tactile sensing in three dimensions.

### DETAILED DESCRIPTION

The following detailed description of certain embodiments presents various descriptions of specific embodiments. However, the innovations described herein can be embodied in a multitude of different ways, for example, as defined and covered by the claims. In this description, reference is made to the drawings where like reference numerals can indicate identical or functionally similar elements. It will be understood that elements illustrated in the figures are not necessarily drawn to scale. Moreover, it will be understood that certain embodiments can include more elements than illustrated in a drawing and/or a subset of the illustrated elements. Further, some embodiments can incorporate any suitable combination of features from two or more drawings. The headings are provided for convenience only and do not impact the scope or meaning of the claims.

### Overview of Tactile Sensing

Tactile sensing is a significant emerging technology for enabling general-purpose robots to interact with physical objects in a manner akin to human dexterity. For robotic dexterous manipulation, the ability to sense and interpret tactile feedback can be significant for performing complex tasks such as grasping delicate objects, adjusting grip force, and interacting with uneven or textured surfaces. Tactile sensing can be used to provide feedback for robotic systems when grasping objects. For example, tactile sensing can be incorporated into humanoid robots. Tactile sensing is also useful for any type of robotic and/or robot system that has the ability to interact with objects in the environment. Specifically, tactile sensing can improve agility for robotic hands or end effectors.

Tactile sensing can also be used to treat patients with paralysis to gain or improve grasping capabilities. For patients with paralysis, tactile sensors can improve hand mobility through a brain machine interface.

Certain robotic systems can lack sufficient tactile feedback, limiting their functionality and versatility. By addressing this gap, tactile sensors can significantly enhance a robot's ability to perceive and respond to its surroundings, paving the way for advancements in robotics for industries such as manufacturing, healthcare, and service. Aspects of this disclosure provide tactile sensing solutions that are well-suited for integration into a variety of applications, including in robotic systems such as for implementing robotic fingers and/or humanoid hands. For example, aspects of this disclosure relate to tactile sensors that include an array of sensor cells to provide high resolution detection of a force. Further aspects provide a tactile sensor that can sense a force in three-dimensions. Yet additional aspects relate to tactile sensors that can detect the proximity of an object in addition to sensing a force applied to the sensor.

There are a variety of types of tactile sensing methods, such as capacitive, piezoresistive, and optical. Magnetic tactile sensing has various advantages over other techniques, including high sensitivity, large dynamic range, high reliability, and fast response. There have been attempts to implement magnetic tactile sensing. However, certain magnetic tactile sensing systems can suffer from magnetic field interference, be difficult to manufacture, and/or may be discrete in nature. Magnetic tactile systems that are vulnerable to magnetic field interference may not be suitable to provide tactile feedback for certain robotic systems. Thus, it is desirable to reduce or eliminate magnetic field interference for magnetic tactile systems.

Aspects of this disclosure relate to magnetic tactile sensing systems and techniques that can address at least some of the above-indicated problems. In some embodiments, the disclosure provides a magnetic tactile sensor that has high spatial resolution, is relatively easy to manufacture, and is substantially immune to distortions from external magnetic fields. Integration can facilitate larger arrays of sensors compared to other techniques. In some embodiments, the disclosed magnetic tactile sensor is capable of 3-dimensional (3D) sensing (e.g., sensing a force in X, Y, and Z direction). Further aspects of this disclosure relate to a coil/sensor configuration or that includes magnets used as a magnetic source. Advantageously, the magnet can be easily manufactured through screen printing in certain applications.

The described magnetic tactile sensor can include a coil and a magnetoresistive (MR) bridge configuration where the sensing is substantially free from external field interference. The configuration of the sensor can ensure that an external magnetic field will generate resistance changes for both resistors of a resistor divider and substantially no change in bridge output.

In embodiments of this disclosure, coils can be used generate magnetic fields of opposite direction for a maximum bridge output and sensitivity. A magnetic shield can further reduce magnetic interference from external magnetic fields. In 3D sensing, a plurality of (e.g., four) coil/MR bridges can move together, so differential from these bridges can represent a magnetic field in the X or Y direction, while the common output can represent the Z direction. Movement of the shields and/or coils can change the magnetic field at the MR sensor, thus providing tactile sensing. Similarly a 3D configuration with magnetic/MR can be configured for maximum magnetic field immunity. Magnet arrays with a same magnetic direction can be manufactured relatively easily.

While coil-AMR, coil-GMR, coil-TMR tactile sensors generally have good direct current (DC) accuracy, they can be sensitive to external magnetic field, especially DC magnetic fields. Manufacturing magnetoresistive (e.g., AMR/GMR/TMR) sensors may also involve special processes. To address these concerns, magnetic tactile sensors can be designed to reduce magnetic interference from external magnetic fields. However, there may be drawbacks to such magnetic tactile sensors in that they may involve relatively complex tactile sensor cell design to reduce the magnetic interference.

In some other embodiments, tactile sensors can be designed based on inductive and/or capacitive coupling. Since the inductive and/or capacitive coupling is based on alternating currents, these sensors may be immune to magnetic interference. For example, inductively coupled sensors can be driven by alternating currents which are typically not sensitive to DC magnetic fields. Another example is a capacitive coupled sensor which can be implemented with a relatively small footprint. Coil-coil tactile sensors can further be enhanced with proximity sensing before contact of an object with the sensor. A coil-coil tactile sensor can also function as a proximity sensor. This can leverage top coil coupling (e.g., inductively and/or capacitively) with a foreign object in proximity.

Advantageously, sensors designed using coil-coil and/or capacitive coupling can be manufactured without the special processes used for magnetoresistive (e.g., AMR/GMR/TMR) sensors. Tactile sensors that use coil-coil and/or capacitive coupling can be designed without a shield and without a bridge detection structure.

Additionally, coil-coil sensors are typically not sensitive to DC magnetic fields, and alternating current immunity is also good when relatively small coil sizes are used. Alternating current immunity can be further enhanced with s-shaped coil designs by cancelling flux from external fields. Coil-coil and capacitive sensors can be used for 3D tactile sensors using a relatively simple design.

In one example, a tactile sensor cell can include a flexible layer, a first coil arranged on a first side of the flexible layer, and a second coil arranged on a second side of the flexible layer. A force applied to the tactile sensor cell is configured to change inductive coupling between the first coil and the second coil. A detection circuit can detect the applied force based on the change in inductive coupling.

In another example, a tactile sensor can include an array of tactile sensor cells, a plurality of pillars, and a detection circuit. A first one of the pillars overlaps a plurality of the tactile sensor cells. The detection circuit is configured to determine a plurality of directional components (e.g., 3 directional components) of a force applied to the first pillar based on outputs of the plurality of tactile sensor cells overlapping the first pillar.

### Example Magnetic Tactile Sensors

Aspects of this disclosure relate to magnetic tactile sensing systems that have high spatial resolution, are relatively easy to manufacture, and are substantially immune to distortions from external magnetic fields. In particular, embodiments of the disclosed technology relate to tactile sensor cells that can be used in an array to form a magnetic tactile sensor.

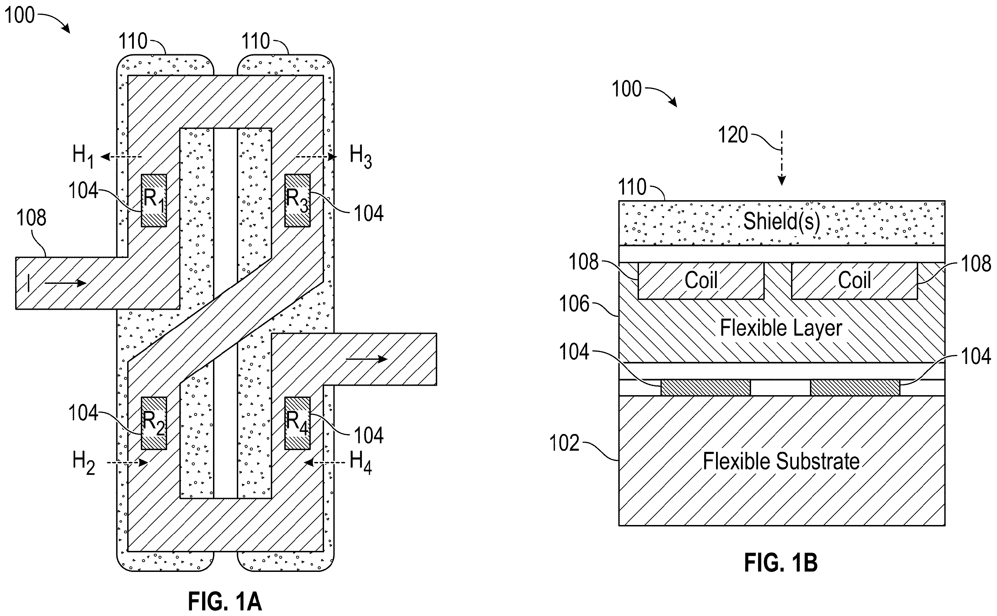

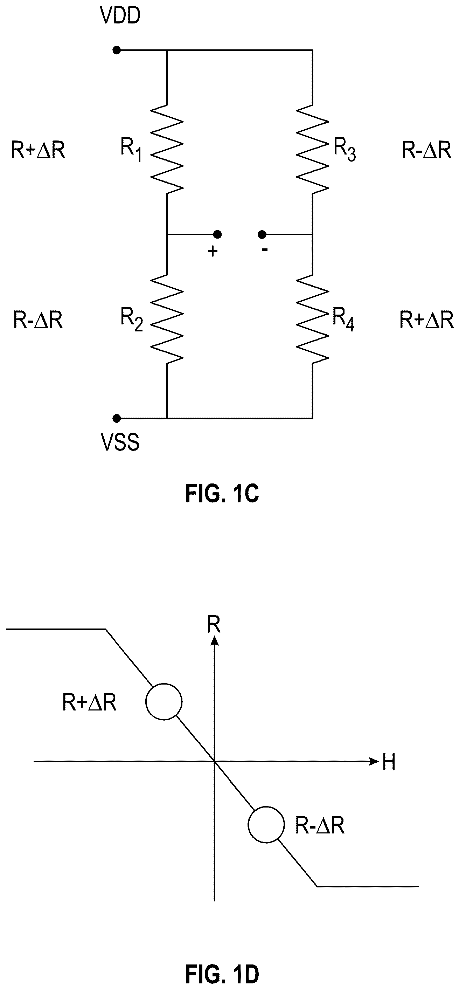

**FIGs. 1A-1D** illustrate an example tactile sensor cell 100 which can be used to create a magnetic tactile sensor in accordance with aspects of this disclosure. In particular, **FIG. 1A** illustrates an embodiment of the layout of the tactile sensor cell 100, **FIG. 1B** illustrates a cross-sectional schematic view of the embodiment of the tactile sensor cell 100, **FIG. 1C** illustrates a circuit showing the connections between the MR sensors, and **FIG. 1D** illustrates a graph of an example output of the tactile sensor. Each of the MR sensors 104 can be referred to as an MR element.

In the illustrated embodiment, the tactile sensor cell 100 includes a substrate 102, a plurality of MR sensors 104 , a flexible layer 106, a coil 108, and one or more magnetic shield(s) 110. In some applications, a magnetic tactile sensor can be implemented without a magnetic shield. As shown in **FIG. 1A****,** the tactile sensor cell 100 includes four MR sensors 104, having resistances labeled R₁, R₂, R₃, and R₄. These MR sensors 104 are arranged in a full bridge configuration as shown in **FIG. 1C****.**

In some embodiments, the substrate 102 can be formed of silicon , glass, or a printed circuit board (PCB). The substrate 102 can be formed of any other suitable material. In some embodiments, the substrate 102 may be a flexible substrate. Depending on the embodiment, the MR sensors 104 can include giant magnetoresistive (GMR), tunnel magnetoresistive (TMR), anisotropic magnetoresistive (AMR) sensors, and/or another suitable MR sensor. The flexible layer 106 may be a deformable layer that can deform in response to the force 120 applied to the tactile sensor cell 100. In some embodiments, the flexible layer 106 can be formed of polydimethylsiloxane (PDMS), which can be spin coated on a wafer or integrated with a PCB. The flexible layer 106 can be an organic polymer layer, PDMS, silicon rubber, or other elastomer(s). The flexible layer 106 can be formed of any other suitable flexible material.

**In** **FIG. 1A****,** the coil 108 is a magnetic field source of the tactile sensor cell 100. The coil 108 can be arranged above the MR sensors 104 to form a path as shown in the layout view of the tactile sensor 100. When a current I is passed through the coil 108, a magnetic field is generated by the coil 108 in the directions H₁, H₂, H₃, and H₄ above the corresponding MR sensors 104. The layout view also shows the shield which can be formed in columns above coil and MR sensors 104. The shield layer 110 can be configured to reduce the magnitude of an external magnetic field. In some embodiments, the shield layer 110 can be formed of NiFe, for example. The shield layer 110 can be formed of any other suitable materials. In some embodiment, the shield layer 110 can function as a concentrator that enhances a magnetic field generated by a coil or other magnetic field source.

As shown in **FIG. 1C****,** the full bridge circuit of the MR sensors 104 can be coupled between two voltage rails (e.g., VDD and VSS) and can provide a differential output. When a force 120 (as shown in **FIG. 1B****)** is applied to the area above the coil 108 and MR sensors 104, the distance between the coil 108 and the MR sensors 104 should change the magnetic field measured at the individual MR sensors 104. This change in magnetic field should result in a change in the resistance R₁-R₄ of the MR sensors 104. Thus, the resistance R₁-R₄ of each of the MR sensors 104 can vary based on the movement of the coil 108 (or other magnetic source) relative to the MR sensors 104. The change in resistance R₁-R₄ for an MR sensor 104 is illustrated by the graph in **FIG. 1D** and may be expressed by Equation (1): $ΔR ∼ I / t$
where t is the thickness of the flexible layer 106. The output of the full bridge can then be expressed as shown in Equation (2): ${V}_{B} = VDD * ΔR / R$

Accordingly, the force 120 can be calculated as shown in Equation (3): $Force ∼ δt ∼ δ \left(I/ΔR\right) ∼ δ \left(I/{V}_{B}\right)$

Due to the physical arrangement of the MR sensors 104, an external magnetic field should have substantially the same effect on the MR sensors 104 having the resistances R₁ and R₂, and substantially the same effect on the MR sensors 104 the resistances R₃ and R₄. In other words, because the external magnetic field should be substantially constant for the entire tactile sensor cell, the resistance R₁-R₄ of each of the MR sensors 104 should vary in the same magnitude and direction in response to the external magnetic field.

Accordingly, the full bridge circuit should cancel out the effects of the external magnetic field, such that the differential output of the full bridge circuit is substantially unaffected by the external magnetic field. Thus, the tactile sensor 100 can be considered to be substantially immune to the external magnetic field. The coil 108 configurations can also be used to reject at least some amount of the external magnetic field.

MR sensors 104 may have an operational window, such that outside of the operational window, measurements may be saturated. By reducing the magnitude of the external magnetic field using the shield and/or the coil 108 configuration, the tactile sensor cell 100 can function within the operational window for a greater range of external magnetic fields.

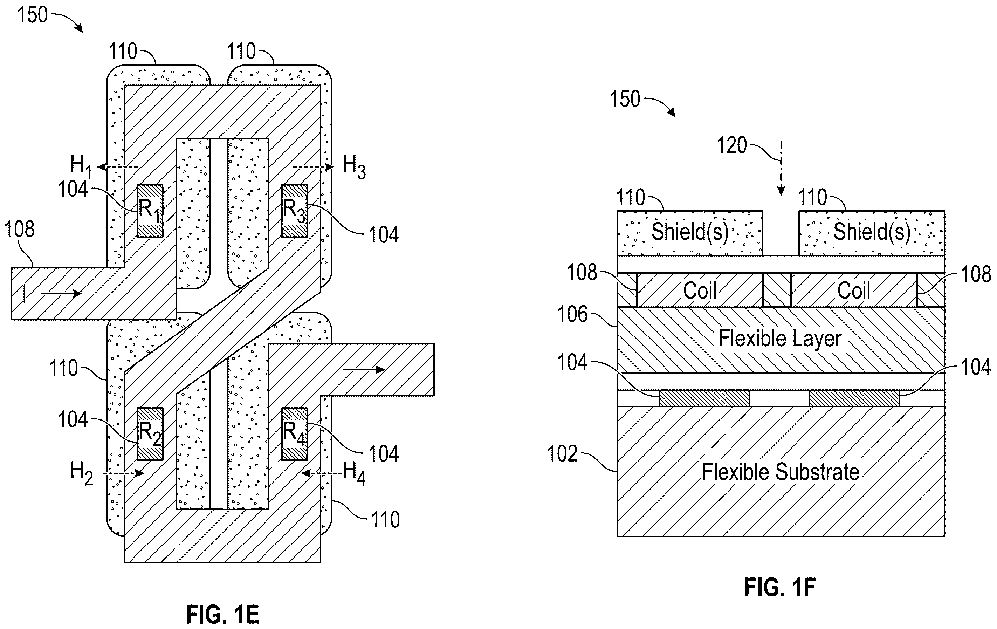

**FIGs. 1E-1F** illustrate another example tactile sensor cell 150 which can be used to create a magnetic tactile sensor in accordance with aspects of this disclosure. In particular, **FIG. 1E** illustrates an embodiment of the layout of the tactile sensor cell 150, **FIG. 1F** illustrates a schematic cross-sectional view of the embodiment of the tactile sensor cell 150. In the example of **FIGs. 1E** and **1F****,** an individual shield 110 is provided for each of the MR sensors 104. This configuration of the shields 110 may be advantageous since the direction for magnetic flux may different between the locations of each of the MR sensors 104.

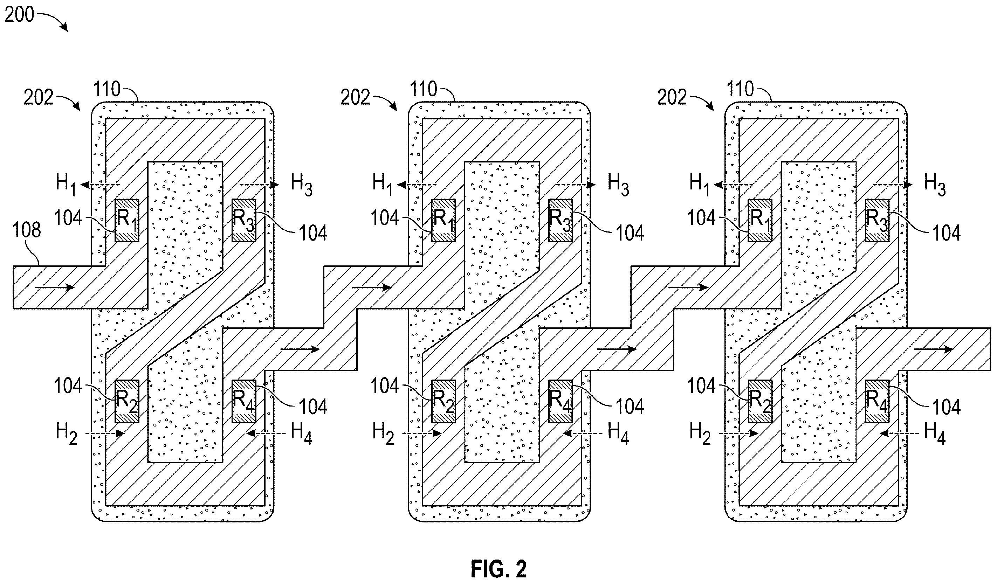

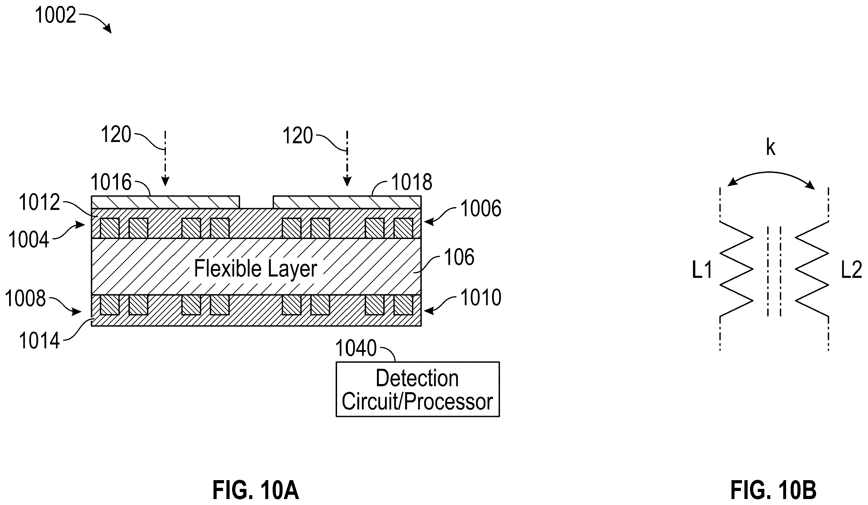

**FIG. 2** illustrates a plurality of tactile sensor cells 202 coupled together to form a tactile sensor 200. In particular, **FIG. 2** shows a row of tactile sensor cells 202 with the coil 108 forming a continuous coil 108 for the row. A continuous coil 108 can simplify physical layout and related circuitry relative to including individual coils 108 for each tactile sensor cell 202. While a single row is shown, multiple rows can be provided to form an array of cells 202. The individual measurements from the cells 202 can be provided to a processor (e.g., such as the detection circuit/processor 1040 of **FIG. 10A****)** to determine the location and depth of any force applied to the array. A detection/circuit processor can be implemented with any of the tactile sensor cells and/or tactile sensors disclosed herein.

The resolution of a magnetic tactile sensor 200 can be increased by providing a larger number of cells 202 in the array. Any suitable number of cells 202 can be arranged into an array without departing from aspects of this disclosure. In one example, a tactile cell 202 may have a size in the range of 100 microns to 3 mm (for example, a side of the tactile cell 202 may have a length from about 100 microns to about 3 mm). In some embodiments, a tactile cell 202 may have a length of up to about 1 mm, with some other embodiments being less than about 100 microns, between about 100 microns to about 1 mm, or larger than about 1 mm. The size of the tactile cell 202 may very depending on the spatial constraints of the sensor array.

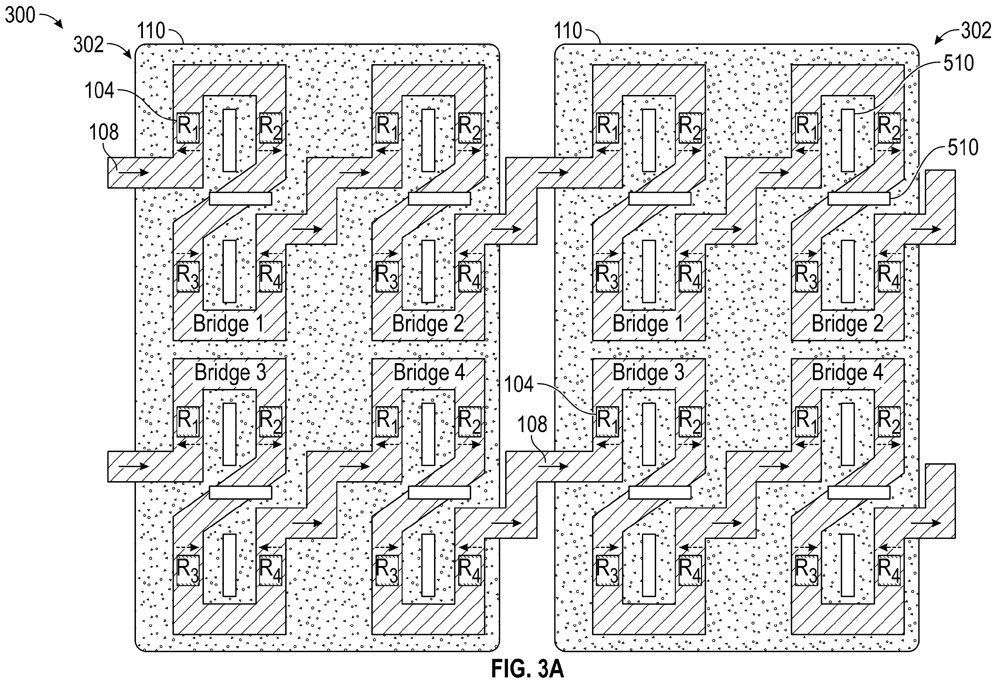

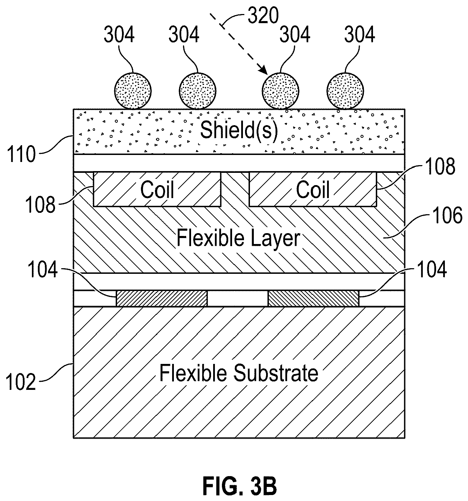

**FIGs. 3A** and **3B** illustrate another tactile sensor 300 formed from a plurality of tactile sensor cells 104 arranged in an array. In particular, **FIG. 3A** illustrates an embodiment of the layout of the tactile sensor 300 and **FIG. 3B** illustrates a schematic cross-sectional view of the embodiment of the tactile sensor 300. As shown in **FIG. 3A****,** four tactile sensor cells Bridge1, Bridge2, Bridge3, and Bridge4 can be arranged together to form a tactile sensor group 302. Each tactile sensor group 302 can include two tactile sensor cells Bridge 1 and Bridge 2 in one row and two tactile sensor cells Bridge 3 and Bridge 4 in another row. Each tactile sensor cell Bridge 1-Bridge 4 can include 4 MR sensors 104. The 4 MR sensors 104 of a tactile sensor cell Bridge 1-Bridge 4 can be arranged as a full bridge, for example, as shown in **FIG. 1C****.** In this embodiment, the differences between the differential measurements by the tactile sensor cells Bridge1-Bridge4 in each group can be used for tactile sensing in three dimensions (e.g., X, Y, and Z).

For example, a force 320 in the Z-direction can result in the coils 108 associated the tactile sensor cells Bridge1-Bridge4 in a group moving closer to the corresponding MR sensors 104. For a force 320 in the X- or Y-direction, the coils 108 move closer to one of the MR sensors 104 and farther away from another of the MR sensors 104 of a given tactile sensor cell Bridge1-Bridge4, depending on the direction of the force 320. A processor can then determine the 3D force 320 based on the difference between the measurement from each of the tactile sensor cells 104 in the tactile sensor cell Bridge1-Bridge4.

In some embodiments, the tactile sensor cells Bridge1-Bridge4 can include bumps 304 that provide friction for lateral movement. This can help improve the sensitivity of the tactile sensors 104 to lateral forces. The bumps 304 can be formed to have any shape (e.g., spherical, ellipsoid, etc.) that improves friction between the tactile sensor system 300 and an object.

The shields 110 in **FIGs. 3A** and **3B** are configured to address the difference in the direction for magnetic flux between the locations of each of the MR sensors 104, similar to the embodiment of **FIG. 1B****.** A common shield can be used for four tactile sensor cells Bridge1-Bridge4. The shields 110 of the sensor cells 302 can include slots 510 in the shield 110. More details regarding the slots 510 are discussed below.

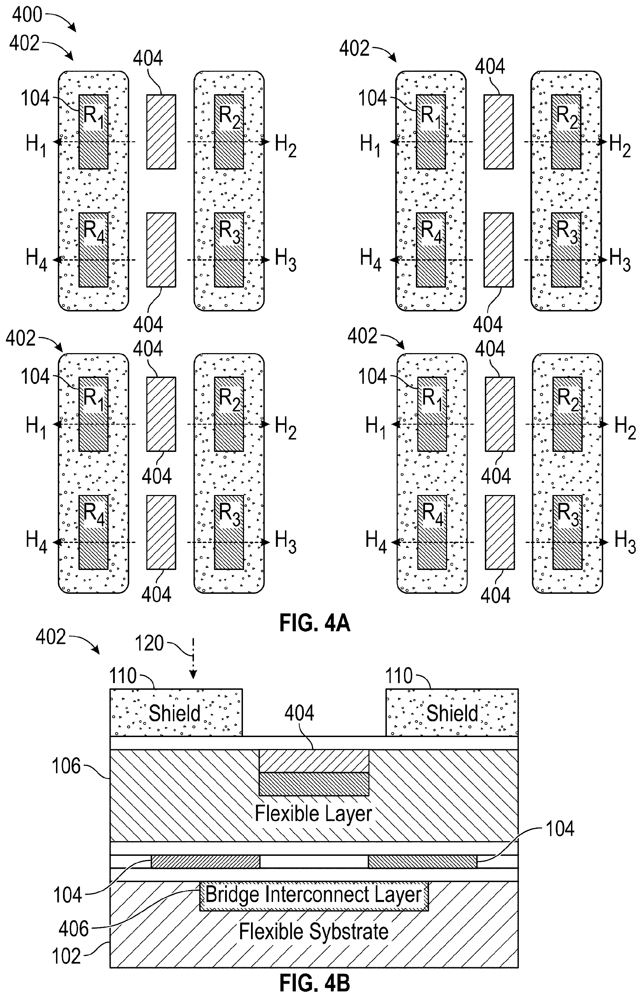

**FIGs. 4A** and **4B** illustrate another embodiment of a tactile sensor cell group 400 in accordance with aspects of this disclosure. In particular, **FIG. 4A** illustrates an embodiment of the layout of the tactile sensor cell group 400 and **FIG. 4B** illustrates a cross-sectional schematic view of a tactile sensor cell forming part of the tactile sensor cell group 400. The magnetic tactile sensor cells 402 of **FIGs. 4A** and **4B** are similar to the embodiment of **FIGs. 1A** and **1B****,** with the coil 108 being replaced by one or more magnets 404. In some embodiments, the magnets 404 can be formed by screen printing. Each of the magnets 404 can be a magnetic field source in the embodiment of **FIGs. 4A** and **4B****.** Although coils 108 and magnets 404 are provided as examples of magnetic field sources, any other suitable magnetic field source can be alternatively or additionally implemented in a tactile sensor in accordance with any suitable principles and advantages disclosed herein. A bridge interconnect layer 406 is also shown in **FIG. 4B****.** The bridge interconnect layer 406 can connect MR sensors 104 in a layer on an opposite side as the flexible layer 106.

To provide a similar external magnetic field immunity effect to the previous embodiments, the MR sensors 104 can have a different physical arrangement in the layout, for example, as shown in **FIG. 4A****.** Thus, the tactile sensor cells 402 can provide substantially the same differential measurement signal from the full bridge as in the embodiments discussed above.

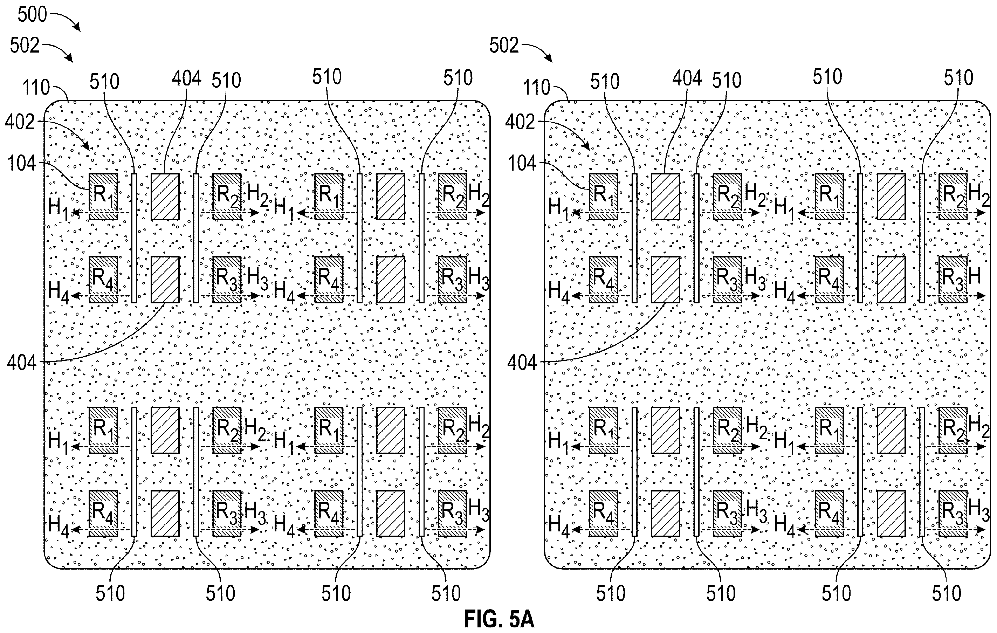

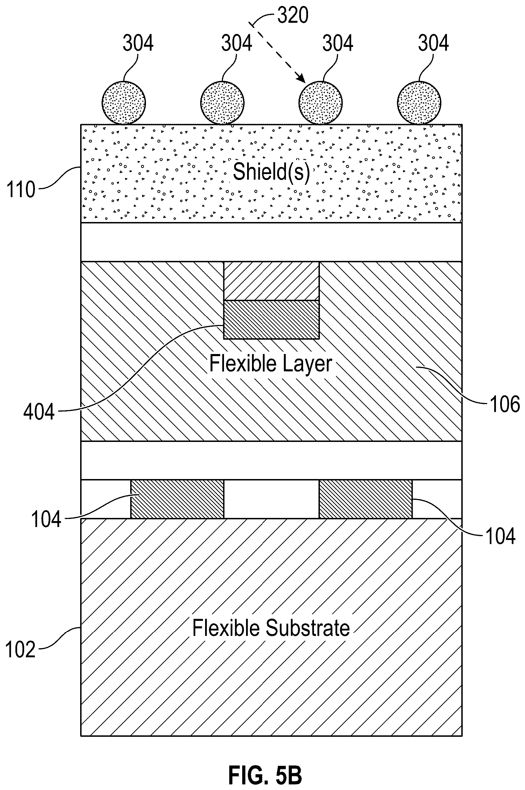

**FIGs. 5A** and **5B** illustrate another embodiment of a tactile sensor 500 formed from a plurality of tactile sensor cells 402 arranged in an array. In particular, **FIG. 5A** illustrates an embodiment of the layout of the tactile sensor 500 and **FIG. 5B** illustrates a cross-sectional schematic view of a tactile sensor cell 502. The embodiment of **FIGs. 5A** and **5B** may be similar to the embodiment of **FIGs. 3A** and **3B****,** using the tactile sensor cells 402 illustrated in **FIGs. 4A** and **4B****.**

As shown in **FIGs. 3A** and **5B****,** the tactile sensor groups 302, 502 can include slots 510 in the shield 110, which can improve the ability of the tactile sensors 300, 500 to reduce the magnitude of the external magnetic field that reaches the MR sensors 104.

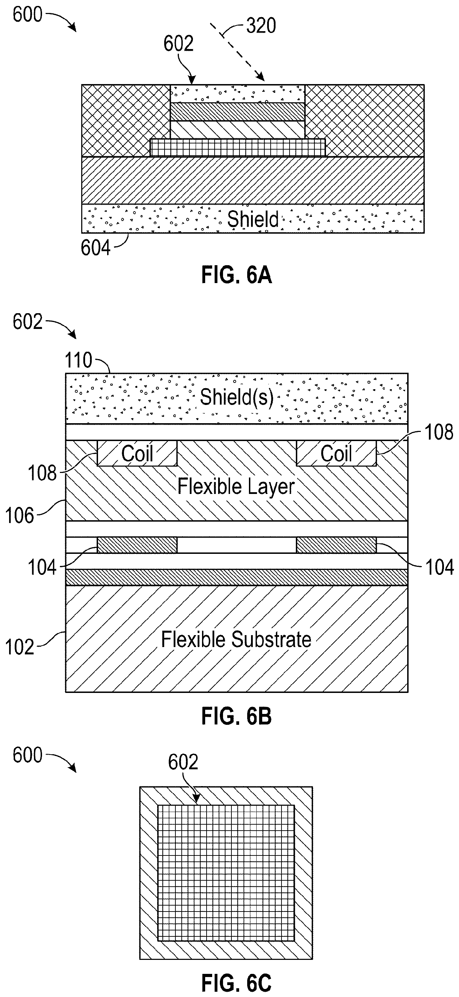

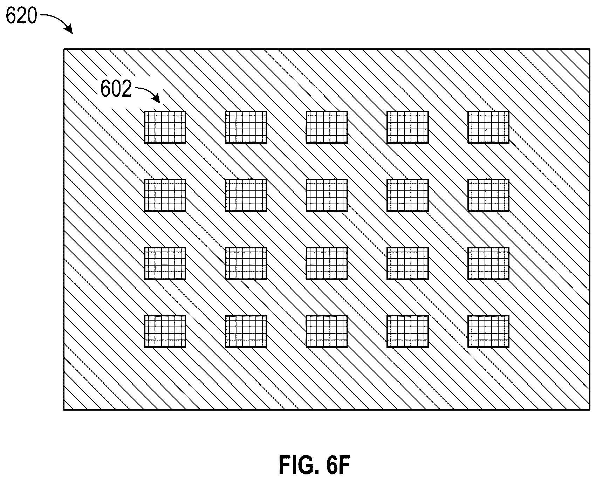

The tactile sensor cells described herein can be scaled to provide magnetic tactile sensors of various sizes. **FIGs. 6A-6C** illustrate an embodiment in which the tactile sensor cells (e.g., an array of MXN tactile sensor cells) can be formed on a single die 602. In particular, **FIGs. 6A** provides a cross-sectional view of the tactile sensor 600 with the single die 602 including an array of tactile sensor cells, **FIG. 6B** provides a cross-sectional view of one of the tactile sensor cells included in the single die 602, and **FIG. 6C** illustrates a plan view of the tactile sensor 600.

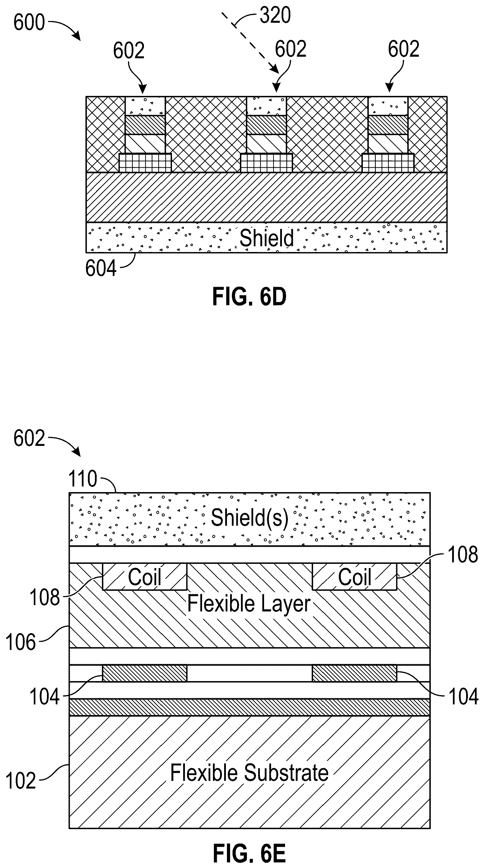

**FIGs. 6D-F** illustrate an embodiment in which the tactile sensor cells 602 can be formed on a MxN array of dies, with each die including one or more tactile sensor cells 602 or one or more tactile sensor groups. **FIG. 6D** provides a cross-sectional view of tactile sensor 620 including a plurality of tactile sensor cell dies 602 each having an individual sensor cell. **FIG. 6E** provides a cross-sectional view of the tactile sensor cell die 602. **FIG. 6C** illustrates a plan view of the tactile sensor 620 including a plurality of tactile sensor cell dies 602.

In some embodiments, the tactile sensor 600, 620 can include a flexible coating, which may be provided on the bottom of the tactile sensor 600, 620. As illustrated, the flexible coating can be in direct contact with the bottom side of the tactile sensor 600, 620. One advantage to using smaller dies 602 as in the tactile sensor 620 is that this may reduce the manufacturing costs. An advantage to the single larger die 602 from the tactile sensor 600 is that it may be simpler to provide a higher resolution sensor 600 (e.g., the distances between the cells can be smaller).

In some embodiments, an additional shield 604 can be included on the bottom of the flexible substrate to provide magnetic shielding from both sides of the magnetic tactile sensor. These dual shields can be added to and/or included with any embodiment disclosed herein.

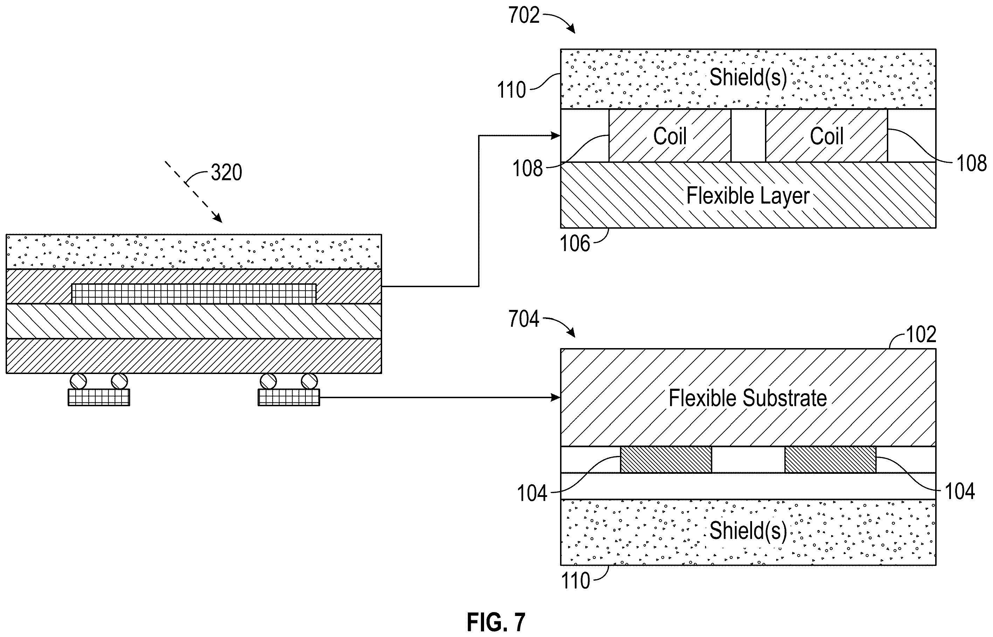

**FIG. 7** illustrates an embodiment in which the coil(s) 108 and MR sensors 104 can be formed on different components 702, 704 and then coupled together. Separately forming the coil(s) 108 and the MR sensors 104 may provide for simpler manufacturing. The coil(s) 108 and MR sensors 104 can be aligned between components 702, 704.

Various embodiments of this disclosure can be manufactured in the form of a fabric that can be applied to any shape. When the magnetic tactile sensor is embodied within a fabric, it may be easier to include the magnetic tactile sensor on applications having different shapes (e.g., on the end effector of a robot) without limiting the shapes of the object onto which the fabric can be applied.

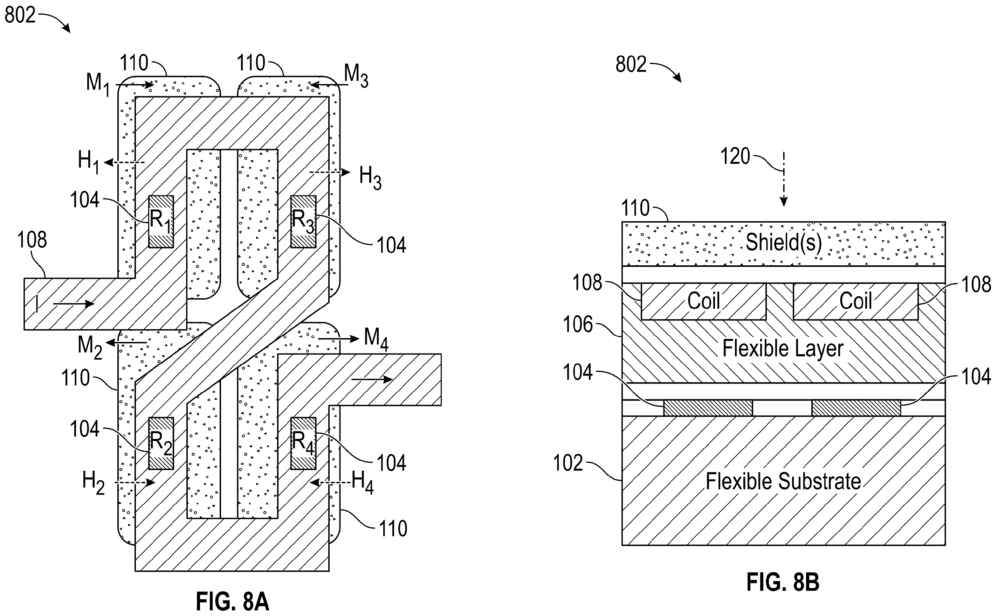

**FIGs. 8A-8H** illustrate additional example tactile sensor cells which can be included in a magnetic tactile sensor in accordance with aspects of this disclosure. The illustrations of **FIGs. 8A-8** **H** are similar to the embodiments of **FIGs. 1A** and **1B****,** with the differences described below. The description of **FIGs. 1A** and **1B** can also apply to any elements that are substantially the same in the embodiments of **FIGs. 8A-8** **H.**

With reference to **FIGs. 8A** and **8B****,** with the coil currents I flowing through the coil 108 can bias the shields 110, resulting in residual (also referred to as remanent) magnetic moments M₁, M₂, M₃, and M₄ in the shields 110 when the coil current I is off. Accordingly, the sensor 802 can work with coil current I switched off. For example, the MR sensors 104 can detect the residual magnetic moments M₁, M₂, M₃, and M₄ in the shields 110 in substantially the same way as detecting the magnetic fields generated by the coil 108 as discussed in the embodiments of **FIGs. 1A** and **1B****.**

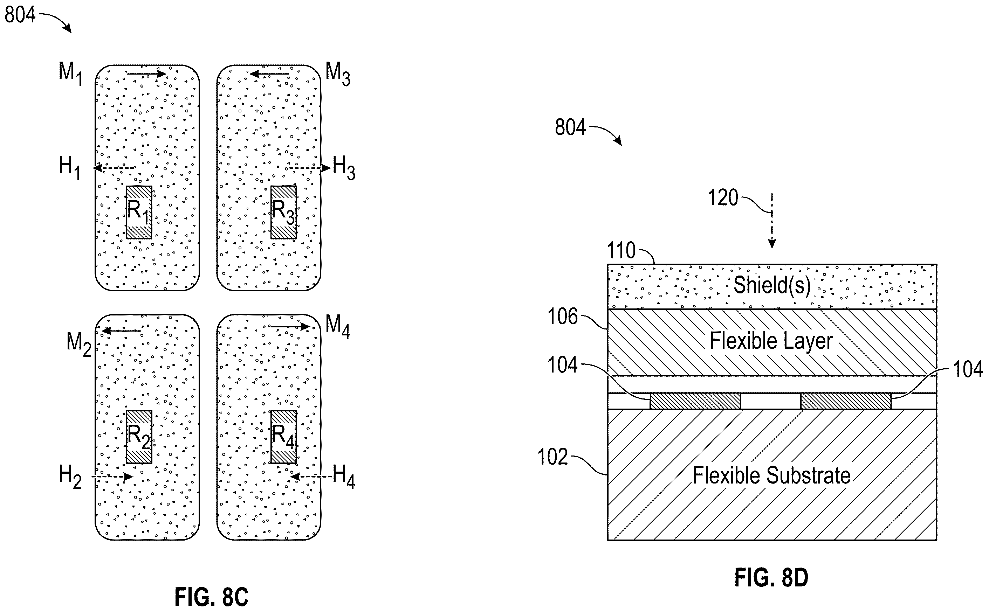

With reference to **FIGs. 8C** and **8D****,** since the tactile sensor cell 804 can work with the coil current I turned off, the shield 110 can be biased in a desired direction during the wafer manufacturing process, so that the tactile sensor cell 804 does not need a coil on the sensor chip to make the sensor 804 work. This is a simplified sensor 804 stack without coil layers as shown. In other words, the biased shields 110 can function as a magnetic field source so that the coil and/or magnets from other embodiments are not used in the embodiment of **FIGs. 8C** and **8D****.**

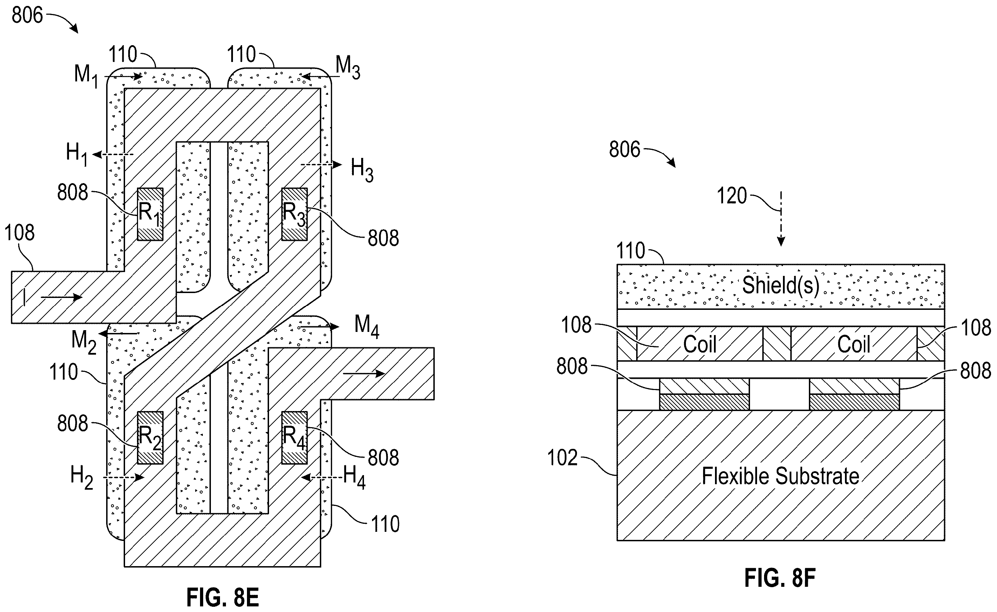

The tactile sensor cell 806 of **FIGs. 8E** and **8F** is similar to the tactile sensor cell 802 of **FIGs. 8A** and **8B****,** with a magnetoelastic layer 808 on top of MR sensor stack. In this embodiment, the tactile sensor cell 806 can be formed without the use of an elastic layer, such as flexible layer 106 of certain other embodiments. In some embodiments, the magnetoelastic layer 808 can be formed of Fe₁₋ₓGaₓ with x=0.2. The applied force 120 can change the magnetization in the magnetoelastic layer 808 and thus the free layer in the MR sensors 104, thereby changing the MR sensor 104 resistance and the bridge or tactile sensor cell output. For example, the force 120 applied to the tactile sensor cell 806 can induce a strain on the magnetoelastic layers 808, with the strain changing the magnetization in the magnetoelastic layer 808. The changing magnetization of the magnetoelastic layer 808 can be measured as a change in the MR sensor 104 resistance.

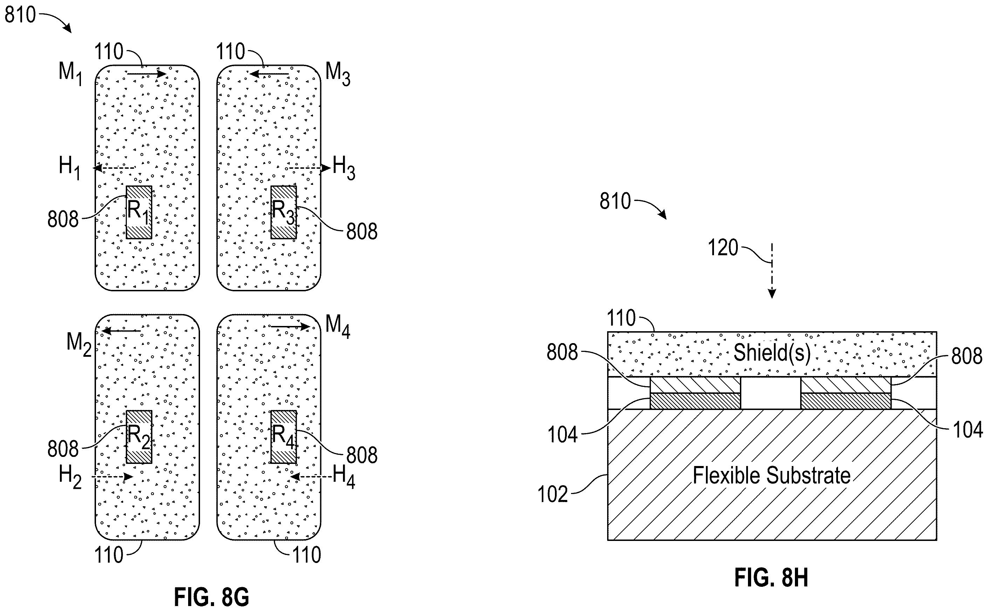

Referring to **FIGs. 8G** and **8H****,** this embodiment is similar to the tactile sensor cell 804 illustrated in **FIGs. 8C** and **8D****,** in that the tactile sensor cell 810 is formed without coils and with magnetoelastic layers on top of the MR sensors 104 (e.g., similar to **FIG. 8E** and **8F****).**

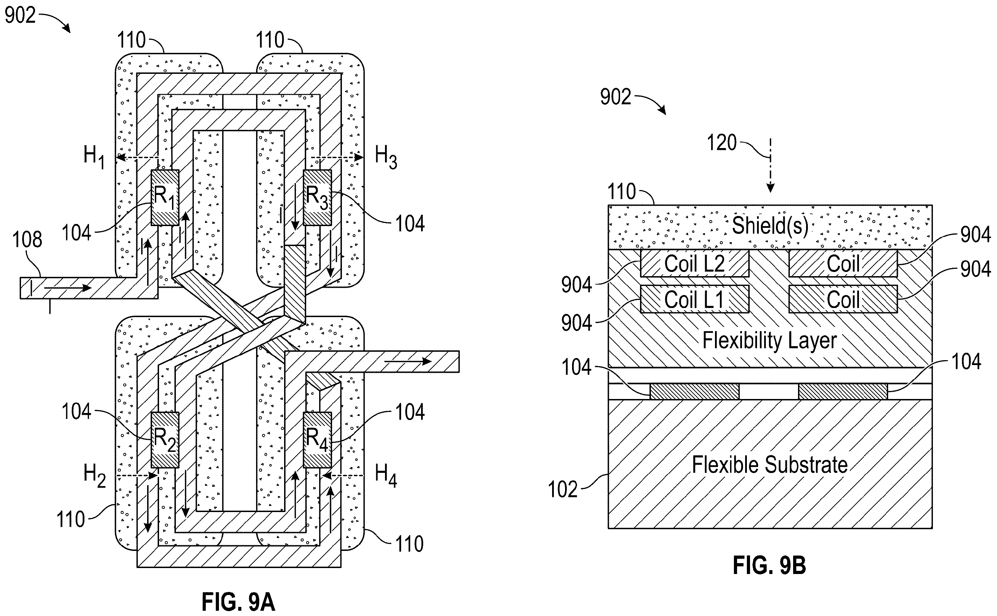

**FIGs. 9A** and **9B** illustrates still another embodiment of a tactile sensor 902 cell having a multi-turn coil 904. In particular, **FIGs. 9A** and **9B** show an embodiment with a coil 904 having two turns. Other embodiments with a coil 904 having a different number of turns (e.g., three or more turns) are also possible.

An additional coil 904 layer can be used to provide bypass, for example, as shown in **FIG. 9A****.** Advantageously, the embodiment of **FIGs. 9A** and **9B** can increase the induced magnetic field for the same amount of current I running through the coil 904 compared to a single coil implementation. Any of the coils disclosed herein can be implemented as a multi-turn coil in certain applications.

### Example Inductive and Capacitive Tactile Sensors

Aspects of this disclosure relate to tactile sensing systems that have high spatial resolution, are relatively easy to manufacture, and are substantially immune to distortions from external magnetic fields. In particular, embodiments of the disclosed technology relate to inductive and capacitive tactile sensor cells that can be used in an array to form a tactile sensor. Such sensor cells can be used for tactile sensing in 3 dimensions in certain applications. In some applications, such sensor cells can implement proximity sensing.

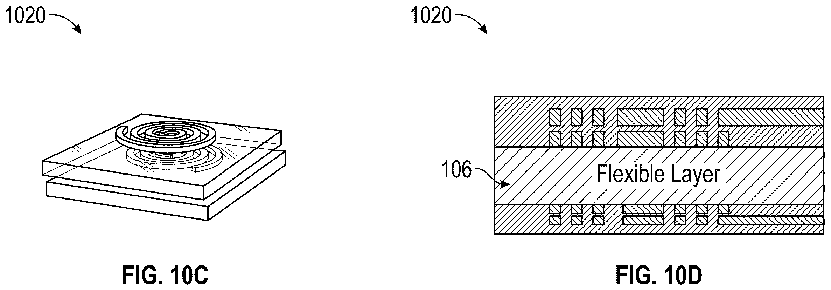

**FIGs. 10A-10D** illustrate an example tactile sensor cell 1002 which can be used to create a tactile sensor in accordance with aspects of this disclosure. **FIG. 10A** includes a cross-sectional view of a first embodiment of the tactile sensor cell 1002. **FIG. 1B** is a diagram illustrating the coupling between coils. **FIG. 10C** provides a perspective view of a second embodiment of a tactile sensor cell. **FIG. 10D** is a cross-sectional view of the second embodiment.

In the illustrated first embodiment of **FIG. 10A****,** the tactile sensor cell 1002 includes a flexible layer 106, a first top coil 1004, a second top coil 1006, a first bottom coil 1008, a second bottom coil 1010, a top dielectric layer 1012, a bottom dielectric layer 1014, a first pillar 1016, and a second pillar 1018. In some embodiments, the flexible layer 106 can be formed of polydimethylsiloxane (PDMS), which can be spin coated on a wafer or integrated with a PCB. The flexible layer 106 can be an organic polymer layer. The flexible layer 106 can be formed of any other suitable flexible material. In some embodiments, the top dielectric layer 1012 and/or the bottom dielectric layer 1014 can include a flexible printed circuit board. Two tactile pixels (also referred to simply as pixels) are included in the illustrated tactile sensor cell 1002.

As an example, the first top coil 1004 can be coupled to the first bottom coil 1008 as shown in **FIG. 10B** illustrating the coupling between coils. The first top coil 1004 and the first bottom coil 1008 can be modelled by first and second inductors L1 and L2, respectively. An alternating current (AC) flowing through one of the first and second inductors L1 and L2 can result in inductive coupling k between the first and second inductors L1 and L2.

In some embodiments, the top coils 1004, 1006 can be independently driven with an AC, provided to each top coil 1004, 1006 independently, and/or the AC can be independently provided to one or more groups of top coils 1004, 1006. In some embodiments, the top coils 1004, 1006 can be driven with an AC together.

The bottom coils 1008, 1010 can be coupled to a detection circuit (or processor) 1040 configured to detect the coupling between the top coils 1004, 1006 and the bottom coils 1008, 1010. In some embodiments, the detection circuit can be configured to detect the coupling for each bottom coil 1008, 1010 separately. The detection circuit can detect a change in an amplitude of a signal generated by an oscillator that includes the bottom coil 1008, 1010. This can detect the applied force 120. The detection circuit 1040 can include any suitable circuitry to process a sensor output (e.g., from any one or more of the tactile sensor cells described herein) and detect a force based on the sensor output. The detection circuit 1040 can be dedicated circuitry or general purpose circuitry programmed to perform processing/detection.

In some embodiments, the distance t between the top coils 1004, 1006 and the bottom coils 1008, 1010 may be the same as the thickness of the flexible layer. When a force is applied to the top of the tactile sensor cell 1002 (e.g., via one or more of the pillars 1016, 1018), the distance t between the top coils 1004, 1006 and the bottom coils 1008, 1010 should decrease. The coupling k between the top and bottom coils 1004-1010 can be a function of the distance t between the top coils 1004, 1006 and the bottom coils 1008, 1010 (e.g., k-f(t)) and can increase when the distance t decreases. Accordingly, the force applied to the tactile sensor cell 1002 can be proportional to a change in the distance t and inversely proportional to a change in the coupling k.

The alternating circuit envelope of the bottom coils 1008, 1010 can be proportional to the coupling with the top coils 1004, 1006. Thus, the detection circuit can be configured to determine the coupling k based on a change in the amplitude of the alternating circuit envelope of the bottom coils 1008, 1010. Advantageously, the coupling between the top and bottom coils 1004-1010 is not typically sensitive to direct current (DC) magnetic fields. Accordingly, the tactile sensor cell 1002 can be substantially immune to distortions from external magnetic fields.

In some cases, the tactile sensor cell 1002 may have a relatively small amount of AC sensitivity. In some embodiments, the tactile sensor cell 1002 can include coils 1004-1010 that are designed to reduce or eliminate AC sensitivity. For example, the coils 1004-1010 can be and/or include S-coils shaped to reduce or eliminate AC sensitivity. However, aspects of this disclosure are not limited thereto and any suitable coil capable of inductive coupling can be used.

In some embodiments, the flexible layer 106 may have a thickness in the range of about 1 µm to 1000 µm or in the range from about 10 µm to 1000 µm. Each of the top and bottom coils 1004-1010 may have a diameter in the range of about 1 µm to 1000 µm in certain embodiments.

As shown in the **FIGs. 10C** and **10D****,** in some embodiments a tactile sensor cell 1020 can include top and/or bottom coils with a plurality of layers (e.g., 2-layers for each coil are illustrated) to increase quality factor and/or efficiency for the top and bottom coils.

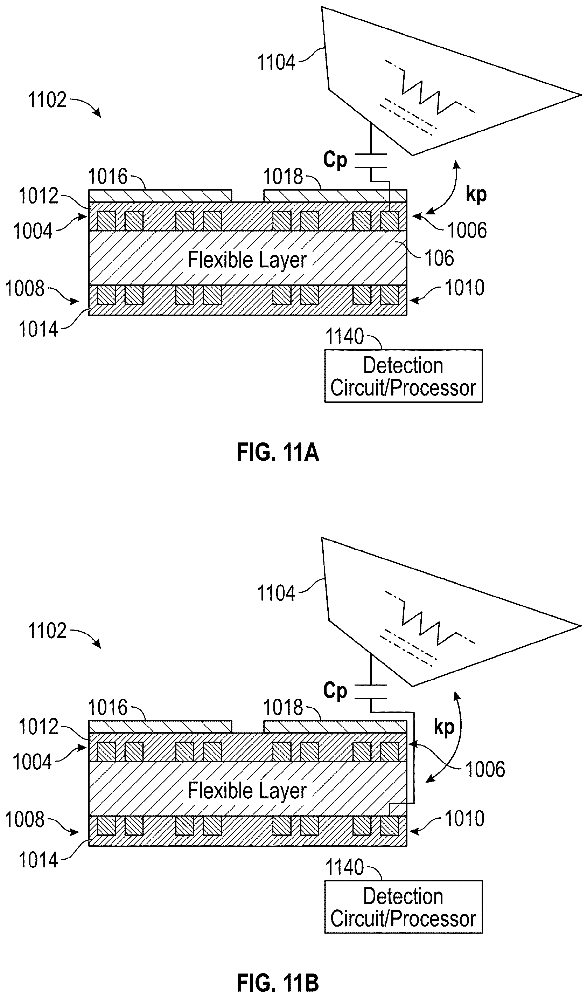

**FIGs. 11A** and **11B** illustrate an example tactile sensor cell 1102 which can be used to create a tactile sensor that can detect proximity of an object 1104 in accordance with aspects of this disclosure. In particular, **FIGs. 11A** and **11B** illustrate a cross-sectional view of the tactile sensor cell 1102 with an object 1104 brought within proximity of the tactile sensor cell 1102. In **FIG. 11A****,** coupling between the object 1104 and one of the top coils 1006 is shown. **FIG. 11B** illustrates coupling between the object 1104 and one of the bottom coils 1010. The embodiment of **FIGs. 11A** and **11B** may be substantially similar to the embodiment of **FIGs. 10A** and **10B****,** in which the detection circuit 1140 is configured to detect the presence of the object 1104. In some embodiments, the detection circuit 1140 can be configured to both detect the force 120 and the presence of the object 1104, and in other embodiments, separate detection circuits 1140 can be provided for detecting the force 120 and for detecting the presence of the object 1104.

With reference to **FIG. 11A****,** when an object 1104 is proximate to the tactile sensor cell 1102, the object 1104 can induce inductive coupling kₚ and capacitive coupling cₚ to one of the top coils 1006. An oscillator amplitude and/or frequency of the top coil 1006 (e.g., the AC signal) can change in response to the inductive coupling kₚ and capacitive coupling cₚ with the object 1104.

With reference to **FIG. 11B****,** when an object 1104 is proximate to the tactile sensor cell 1102, the object 1104 can induce inductive coupling kₚ and capacitive coupling cₚ to one of the bottom coils 1010. The amplitude and/or frequency of the bottom coil 1010 can change in response to the inductive coupling kₚ and capacitive coupling cₚ with the object 1104.

Since the amplitude and/or frequency of the bottom coil 1010 can change due to the inductive coupling kₚ and/or capacitive coupling cₚ between the object 1104 and one or both of the top coil 1006 and bottom coil 1010, the proximity of the object 1104 can be detected by a detection circuit that measures these changes to the AC signal induced in the bottom coil 1010. The proximity of an object 1104 can reduce the amplitude of the AC signal in the bottom coil 1010 while a force applied to the tactile sensor cell 1102 can increase coupling and amplitude. Thus, the detection circuit can distinguish between the proximity of an object 1104 and a force applied to the tactile sensor cell 1104. Larger objects 1104 can result in inductive coupling kₚ and/or capacitive coupling cₚ with a plurality of top and/or bottom coils 1004-1010, such that a coil array can detect changes due to coupling with the plurality of coils 1004-1010. In some embodiments, metal objects 1104 may induce relatively larger inductive coupling kₚ and non-metal objects 1104 may induce relatively larger inductive coupling cₚ.

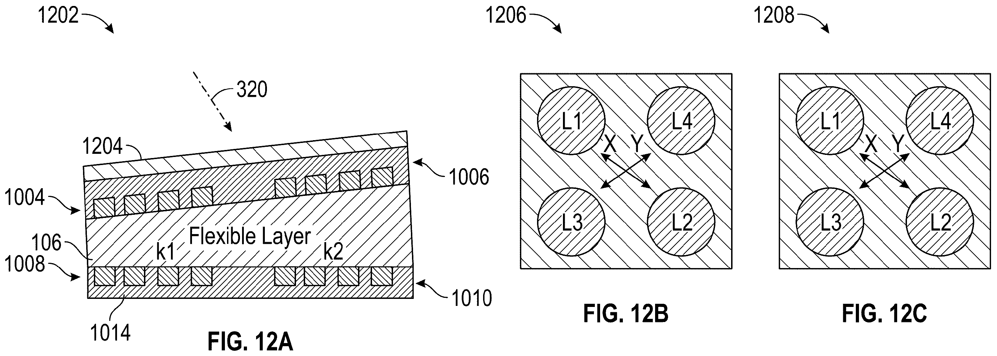

**FIGs. 12A-12C** illustrate another tactile sensor cell 1202 which can be used to create a tactile sensor configured to be used for tactile sensing in three dimensions (e.g., X, Y, and Z). In particular, **FIG. 12A** includes a cross-section view of the tactile sensor cell 1202, **FIG. 12B** is a view of the top coils 1206 of a three-dimensional pixel, and **FIG. 12C** provides a view of the bottom coils 1208 of the three-dimensional pixel. Together, the top coils 1206 and the bottom coils 1208 form a three-dimensional pixel 1206, 1208.

The tactile sensor cell 1202 of **FIGs. 12A-12C** is similar to the embodiment of **FIGs. 10A-10D****,** with a single pillar 1204 shared by and overlapping with a plurality of top and bottom coils 1004-1010. As shown in **FIG. 12A****,** a force 320 applied to the pillar 1204 can result in different distances t between the pairs of top and bottom coils 1004-1010 overlapping the pillar 1204. Thus, when the force 320 includes X and/or Y components, the pillar 1204 may be tilted, resulting in these different distances t. In the illustrated example with the force 320, the inductive coupling k₁ should increase while the inductive coupling k₂ should decrease.

As shown in **FIGs. 12B** and **12C****,** the three-dimensional pixels 1206, 1208 can be arranged such that two of the coils (L1 and L2) are aligned in the X direction and two of the coils (L3 and L4) are aligned in the Y direction. In the event that the cross-section of **FIG. 12A** is taken along the X-direction, the coils L1 and L2 of top portion of the three-dimensional pixel 1206 respectively correspond to the top coil 1004 and the top coil 1006. Similarly, the coils L1 and L2 of the bottom portion of the three-dimensional pixel 1208 respectively correspond to the bottom coil 1008 and the bottom coil 1010. The difference between the inductive coupling k for pixels in the X and Y directions can be combined with the Z direction sensing described in connection with the embodiment of **FIGs. 10A-10D** to detect the force 320 in three dimensions. In some embodiments, the Z direction component of the force 320 can be determined based on an average of the inductive coupling for the coils included in the pixels 1206, 1208.

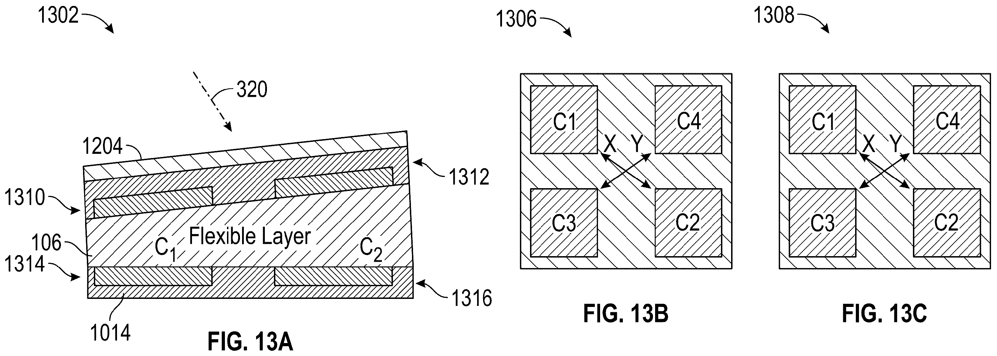

**FIGs. 13A-13C** illustrate another tactile sensor cell 1302 which can be used to create a tactile sensor configured to be used for tactile sensing in three dimensions. In particular, **FIG. 13A** includes a cross-section view of the tactile sensor cell 1302, **FIG. 13B** a view of the top plates 1306 of a three-dimensional pixel, and **FIG. 13C** is a view of the bottom plates 1308 of a three-dimensional pixel. Together, the top plates 1306 and the bottom plates 1308 form a three-dimensional pixel 1306, 1308.

The tactile sensor cell 1302 of **FIGs. 13A-13C** is similar to the embodiment of **FIGs. 12A-12C****,** with a plurality of plates 1310, 1312, 1314, 1316 in place of the plurality of top and bottom coils 1004-1010. As shown in **FIG. 13A****,** a three dimensional force 320 applied to the pillar 1204 can result in different distances t between the pairs of top and bottom plates 1310-1316 overlapping the pillar 1204. Thus, when the force 320 includes X and/or Y components, the pillar 320 may be tilted, resulting in these different distances t. In the illustrated example with the force 320, the capacitive coupling c₁ should increase while the capacitive coupling c₂ should decrease when the force 320 is applied.

The three-dimensional pixels 1306, 1308 can be arranged such that two of the pairs of plates (C1 and C2) are aligned in the X direction and two of the pairs of plates (C3 and C4) are arranged in the Y direction. In the event that the cross-section of **FIG. 13A** is taken along the X-direction, the plates C1 and C2 of top portion of the three-dimensional pixel 1306 respectively correspond to the top plate 1310 and the top plate 1312. Similarly, the plates C1 and C2 of the bottom portion of the three-dimensional pixel 1308 respectively correspond to the bottom plate 1314 and the bottom plate 1316. The difference between the capacitive coupling c for pixels in the X and Y directions can be combined with the Z direction sensing similar to the Z direction sensing described in connection with the embodiment of **FIGs. 10A-10D** as applied to capacitive sensing to detect a three-dimensional force 320. In some embodiments, the Z direction component of the force can be determined based on an average of the capacitive coupling for the plates 1310-1316 included in the pixel 1306, 1308.

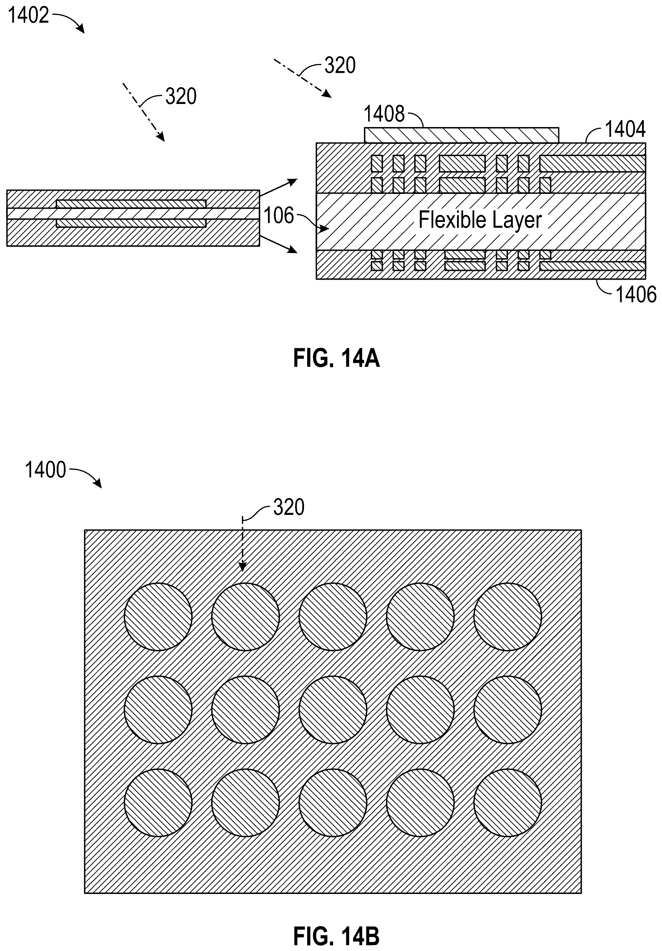

**FIGs. 14A-14B** illustrate another tactile sensor cell 1402 which can be used to create a tactile sensor configured to be used for tactile sensing in three dimensions. In particular, **FIG. 14A** includes a cross-section views of the tactile sensor cell 1402 and **FIG. 14B** is a view of a tactile sensor 1400 including an array of tactile sensor cells 1402.

The tactile sensor cell 1402 of **FIG. 14A** includes a top flexible PCB 1404 and a bottom flexible PCB 1406 with a flexible layer 106 in between. The top flexible PCB 1404, the bottom flexible PCB 1406, and the flexible layer 106 can be molded together as a package.

In some embodiments, one pillar 1408 can be provided for a group of coils or capacitor plates. The pillar 1408 is sized to ensure that the coils for each plate move together. For example, the pillar 1408 may have substantially the same area as the groups of coils overlapping the pillar 1408. This ensures that a force applied to the pillar 1408 results in movement of substantially the entirety of the overlapping coil rather than movement of only a portion of the coil. In the embodiment of **FIGs. 14A** and **14B****,** the pillar 1408 formed over a group of coils or capacitor plates can enable the force 320, which can represent a touch with each group of coils/plates able to move independently.

With reference to **FIGs. 13A-13C** and **14A-14B,** a tactile sensor 1400 configured to sense a force 320 in three dimensions can include an array of tactile sensor cells 1302, 1402, a plurality of pillars 1204, 1408, and a detection circuit. Each of the pillars 1204, 1408 can overlap a plurality of the tactile sensor cells 1302, 1402. The detection circuit can be configured to determine a plurality of directional components (e.g., the X, Y, and Z components) of a force 320 applied to a first one of the pillars 1204, 1408 based on outputs of the plurality of tactile sensor cells 1302, 1402 overlapping the first pillar 1204, 1408. In some other applications, one pillar 1204, 1408 can overlap with one tactile sensor cell 1302, 1402 and a detection circuit can determine a plurality of direction components of force based on outputs from the tactile sensor cell 1302, 1402.

In some embodiments, the same detection circuit can be used to detect both a force (including a 3D force) applied to the tactile sensor and proximity of an object to the tactile sensor. In some other embodiments, separate detection circuits can be used for detecting force and object proximity.

### Applications, Terminology, and Conclusion

Tactile sensing systems (e.g., magnetic tactile sensing systems and/or 3D tactile sensing systems) disclosed herein can be implemented in any suitable application that can benefit from tactile sensing. Example applications include, but are not limited to, robotics and treating paralysis.

In the embodiments described above, sensors, circuits, systems, and methods for tactile sensing are described in connection with particular embodiments. It will be understood, however, that the principles and advantages of the embodiments can be used for any other suitable sensors, circuits, systems, and methods with a tactile sensing system.

Unless the context clearly requires otherwise, throughout the description and the claims, the words "comprise," "comprising," "include," "including," and the like are to be construed in an inclusive sense, as opposed to an exclusive or exhaustive sense; that is to say, in the sense of "including, but not limited to." The words "coupled" or connected", as generally used herein, refer to two or more elements that may be either directly connected, or connected by way of one or more intermediate elements. Thus, although the various schematics shown in the figures depict example arrangements of elements and components, additional intervening elements, devices, features, or components may be present in an actual embodiment (assuming that the functionality of the depicted circuits is not adversely affected). Additionally, the words "herein," "above," "below," and words of similar import, when used in this application, shall refer to this application as a whole and not to any particular portions of this application. Where the context permits, words in the Detailed Description using the singular or plural number may also include the plural or singular number, respectively. The words "or" in reference to a list of two or more items, is intended to cover all of the following interpretations of the word: any of the items in the list, all of the items in the list, and any combination of the items in the list. All numerical values provided herein are intended to include similar values within a measurement error.

Moreover, conditional language used herein, such as, among others, "can," "could," "might," "may," "e.g.," "for example," "such as" and the like, unless specifically stated otherwise, or otherwise understood within the context as used, is generally intended to convey that certain embodiments include, while other embodiments do not include, certain features, elements and/or states.

The teachings of the embodiments provided herein can be applied to other systems, not necessarily the systems described above. The elements and acts of the various embodiments described above can be combined to provide further embodiments. The acts of the methods discussed herein can be performed in any order as appropriate. Moreover, the acts of the methods discussed herein can be performed serially or in parallel, as appropriate.

While certain embodiments of the inventions have been described, these embodiments have been presented by way of example only, and are not intended to limit the scope of the disclosure. Indeed, the novel circuits, methods, and systems described herein may be embodied in a variety of other forms. Furthermore, various omissions, substitutions and changes in the form of the circuits, methods, apparatus and systems described herein may be made without departing from the spirit of the disclosure. For example, while the disclosed embodiments are presented in given arrangements, alternative embodiments may perform similar functionalities with different components and/or circuit topologies, and some elements may be deleted, moved, added, subdivided, combined, and/or modified. Each of these elements may be implemented in a variety of different ways. Any suitable combination of the elements and acts of the various embodiments described above can be combined to provide further embodiments. The accompanying claims and their equivalents are intended to cover such forms or modifications as would fall within the scope and spirit of the disclosure. Accordingly, the scope of the present inventions is defined by reference to the claims.

Although the claims presented here are in single dependency format for filing at the USPTO, it is to be understood that any claim may depend on any preceding claim of the same type except when that is clearly not technically feasible.

### Aspects of the disclosure

Non-limiting aspects of the disclosure are set out in the following numbered clauses:
1. A force sensor, comprising:
   an array of force sensor cells;
   a plurality of pillars, a first pillar of the plurality of pillars overlapping a force sensor cell of the force sensor cells of the array; and
   a detection circuit configured to determine a plurality of directional components of a force applied to the first pillar based on output of the force sensor cell.
2. The force sensor of clause 1, wherein the force sensor cell includes:
   a flexible layer;
   a first coil arranged on a first side of the flexible layer; and
   a second coil arranged on a second side of the flexible layer,
   wherein a force applied to the first pillar is configured to change inductive coupling between the first coil and the second coil.
3. The force sensor of any preceding clause, wherein the force sensor cell includes:
   a flexible layer;
   a first capacitor plate arranged on a first side of the flexible layer; and
   a second capacitor arranged on a second side of the flexible layer,
   wherein a force applied to the first pillar is configured to change capacitive coupling between the first capacitor plate and the second capacitor plate.
4. The force sensor of any preceding clause, wherein the detection circuit is configured to determine a first directional component of the force perpendicular to a plane of the force sensor based on an average of the outputs of the force sensor cell.
5. The force sensor of any preceding clause, wherein the detection circuit is configured to determine a first directional component of the force parallel to a plane of the force sensor based on a difference in the outputs from at least two sensors of the force sensor cell.
6. The force sensor of any preceding clause, wherein the plurality of directional components comprise three dimensions of the force.
7. A robot comprising the force sensor of any preceding clause and an end effector, wherein the force sensor is configured for force sensing on the end effector.
8. A force sensor cell, comprising:
   a flexible layer;
   a first coil arranged on a first side of the flexible layer; and
   a second coil arranged on a second side of the flexible layer, the second side being opposite to the first side,
   wherein a force applied to the force sensor cell is configured to change inductive coupling between the first coil and the second coil.
9. The force sensor cell of clause 8, wherein the first coil is configured to be driven with a first alternating current, and a second alternating current is induced in the second coil due to the inductive coupling with the first alternating current in the first coil.
10. The force sensor cell of clause 9, wherein the force is configured to change an amplitude of the second alternating current induced in the second coil.
11. The force sensor cell of any of clauses 8 to 10, wherein each of the first coil and the second coil is formed with a plurality of layers.
12. The force sensor cell of any of clauses 8 to 11, wherein the force sensor cell is configured such that an object proximate to the force sensor cell induces a coupling with at least one of the first coil or the second coil, and the coupling changes a parameter of an alternating current of the second coil.
13. The force sensor cell of any of clauses 8 to 12, further comprising a pillar overlapping the first coil and the second coil, the force being applied to the pillar.
14. The force sensor cell of any of clauses 8 to 13, wherein:
   the force applied to the force sensor cell is configured to increase an amplitude of an alternating current of the second coil, and
   an object proximate to the force sensor cell is configured to reduce the amplitude of the alternating current of the second coil.
15. A force sensor, comprising:
   the force sensor cell of any of clauses 8 to 14; and
   a detection circuit configured to detect the change in the inductive coupling between the first coil and the second coil.
16. A robot comprising the force sensor cell of any of clauses 8 to 14 and an end effector, wherein the force sensor cell is configured for force sensing on the end effector.
17. A robot comprising the force sensor of clause 15 and an end effector, wherein the force sensor is configured for force sensing on the end effector.
18. A method of force sensing, comprising:
   providing a force sensor cell comprising a flexible layer, a first coil, and a second coil on an opposite side of the flexible layer than the first coil; and
   detecting force applied to the force sensor cell based on a change in inductive coupling between the first coil and the second coil.
19. The method of clause 18, further comprising:
   driving the first coil with a first alternating current; and
   detecting a second alternating current induced in the second coil due to the inductive coupling with the first alternating current in the first coil.
20. The method of clause 19, wherein the force change an amplitude of the second alternating current induced in the second coil.
21. The method of any of clauses 18 to 20, wherein the force sensor is integrated with an end effector of a robot, and the robot performs the detecting.

## Claims

1. A force sensor, comprising:
an array of force sensor cells;
a plurality of pillars, a first pillar of the plurality of pillars overlapping a force sensor cell of the force sensor cells of the array; and
a detection circuit configured to determine a plurality of directional components of a force applied to the first pillar based on output of the force sensor cell.

2. The force sensor of claim 1, wherein the force sensor cell includes:
a flexible layer;
a first coil arranged on a first side of the flexible layer; and
a second coil arranged on a second side of the flexible layer,
wherein a force applied to the first pillar is configured to change inductive coupling between the first coil and the second coil.

3. The force sensor of any preceding claim, wherein the force sensor cell includes:
a flexible layer;
a first capacitor plate arranged on a first side of the flexible layer; and
a second capacitor arranged on a second side of the flexible layer,
wherein a force applied to the first pillar is configured to change capacitive coupling between the first capacitor plate and the second capacitor plate.

4. The force sensor of any preceding claim, wherein the detection circuit is configured to determine a first directional component of the force perpendicular to a plane of the force sensor based on an average of the outputs of the force sensor cell.

5. The force sensor of any preceding claim, wherein the detection circuit is configured to determine a first directional component of the force parallel to a plane of the force sensor based on a difference in the outputs from at least two sensors of the force sensor cell.

6. The force sensor of any preceding claim, wherein the plurality of directional components comprise three dimensions of the force.

7. A robot comprising the force sensor of any preceding claim and an end effector, wherein the force sensor is configured for force sensing on the end effector.

8. A force sensor cell, comprising:
a flexible layer;
a first coil arranged on a first side of the flexible layer; and
a second coil arranged on a second side of the flexible layer, the second side being opposite to the first side,
wherein a force applied to the force sensor cell is configured to change inductive coupling between the first coil and the second coil.

9. The force sensor cell of claim 8, wherein the first coil is configured to be driven with a first alternating current, and a second alternating current is induced in the second coil due to the inductive coupling with the first alternating current in the first coil, and
optionally, wherein the force is configured to change an amplitude of the second alternating current induced in the second coil.

10. The force sensor cell of claim 8 or claim 9, wherein at least one of the following applies:
(a) each of the first coil and the second coil is formed with a plurality of layers;
(b) the force sensor cell is configured such that an object proximate to the force sensor cell induces a coupling with at least one of the first coil or the second coil, and the coupling changes a parameter of an alternating current of the second coil;
(c) the force applied to the force sensor cell is configured to increase an amplitude of an alternating current of the second coil, and
an object proximate to the force sensor cell is configured to reduce the amplitude of the alternating current of the second coil.

11. The force sensor cell of any of claims 8 to 10, further comprising a pillar overlapping the first coil and the second coil, the force being applied to the pillar.

12. A method of force sensing, comprising:
providing a force sensor cell comprising a flexible layer, a first coil, and a second coil on an opposite side of the flexible layer than the first coil; and
detecting force applied to the force sensor cell based on a change in inductive coupling between the first coil and the second coil.

13. The method of claim 12, further comprising:
driving the first coil with a first alternating current; and
detecting a second alternating current induced in the second coil due to the inductive coupling with the first alternating current in the first coil.

14. The method of claim 13, wherein the force change an amplitude of the second alternating current induced in the second coil.

15. The method of any of claims 12 to 14, wherein the force sensor is integrated with an end effector of a robot, and the robot performs the detecting.
